# EUROPEAN PATENT APPLICATION

(11) **EP 4 600 638 A1**
(43) Date of publication of application: **13.08.2025**
(21) Application number: 23891493.1
(22) Date of filing: 10.11.2023
(51) Int. Cl.: G01N 27/22, G01N 27/24

(54) **MOLDING QUALITY EVALUATION METHOD, MOLDED PRODUCT INSPECTION METHOD, MOLDING QUALITY EVALUATION DEVICE, AND MOLDED PRODUCT INSPECTION DEVICE**

(30) Priority: 14.11.2022 JP 2022181772
(71) Applicant: Akita University, Akita-shi Akita 010-8502 (JP); Mitsubishi Heavy Industries, Ltd., Tokyo 100-8332 (JP)
(72) Inventor: MURAOKA, Mikio, Akita-shi, Akita 010-8502 (JP); YAMAGUCHI, Makoto, Akita-shi, Akita 010-8502 (JP); KAMIHARA, Nobuyuki, Tokyo 100-8332 (JP); TAKAGI, Kiyoka, Tokyo 100-8332 (JP); MATSUYAMA, Daiki, Tokyo 100-8332 (JP); KAMO, Sota, Tokyo 100-8332 (JP)
(74) Representative: Hoffmann Eitle
(86) International application number: PCT/JP2023/040617
(87) International publication number: WO 2024/106345

(57) **Abstract**

A molding quality evaluation method for evaluating molding quality of a molded product containing resin includes the steps of: with a state of the molded product before molding being set as a reference state, acquiring as a reference value a capacitance value of the molded product in the reference state; with a state of the molded product during molding being set as a molding state developed over time from the reference state, acquiring as a measurement value the capacitance value of the molded product in the molding state; deriving as a measurement evaluation value a value obtained by non-dimensionalizing the measurement value with the reference value; and evaluating the molding quality based on the measurement evaluation value.

## Description

### Field

The present disclosure relates to a molding quality evaluation method, a molded product inspection method, a molding quality evaluation device, and a molded product inspection device.

### Background

In the related art, a joint section evaluation method is known to inspect weak bond bonding by a nondestructive method to evaluate the bonding state of a bonding section (for example, see Patent Literature 1). In this joint section evaluation method, an AC signal is applied to the bonding section, and from current and voltage values obtained by varying the frequency of the AC signal and measuring current and voltage, evaluation values concerning predetermined electrical characteristics are derived.

### Citation List

### Patent Literature

Patent Literature 1: Japanese Patent Application Laid-open No. 2017-83300 Summary

### Technical Problem

However, in the joint section evaluation method of Patent Literature 1, since the frequency of the applied AC signal is high, an error factor resulting from the difference in the shape of the bonding section is large. For example, when the thickness of the bonding section differs by several tens of micrometers or the shape varies by millimeters as a shape error, it becomes difficult to detect an appropriate evaluation value due to an adverse effect of a signal-to-noise ratio (S/N). Another evaluation method being considered is to heat the bonding section after molding to evaluate molding quality; however, in this case, the molding quality of the bonding section may be affected.

Therefore, an object of the present disclosure is to provide a molding quality evaluation method, a molded product inspection method, a molding quality evaluation device, and a molded product inspection device that can suitably evaluate molding quality of a molded product without affecting the molding quality.

### Solution to Problem

A molding quality evaluation method according to the present disclosure is for evaluating molding quality of a molded product containing resin, and includes the steps of: with a state of the molded product before molding being set as a reference state, acquiring as a reference value a capacitance value of the molded product in the reference state; with a state of the molded product during molding being set as a molding state developed over time from the reference state, acquiring as a measurement value the capacitance value of the molded product in the molding state; deriving as a measurement evaluation value a value obtained by non-dimensionalizing the measurement value with the reference value; and evaluating the molding quality based on the measurement evaluation value.

A molded product inspection method according to the present disclosure is for inspecting the molding quality of the molded product based on an evaluation result of the above-described molding quality evaluation method, a strength evaluation value satisfying a required strength of the molded product being acquired in advance. The molded product inspection method includes the steps of: acquiring a sound evaluation value of the molded product whose molding quality has been previously evaluated to be sound; acquiring a defective evaluation value of the molded product whose molding quality has been previously evaluated to be defective; acquiring the measurement evaluation value derived in the molding quality evaluation method; and determining whether an inspection has been passed, based on the acquired measurement evaluation value and strength evaluation value. The strength evaluation value is set between the sound evaluation value and the defective evaluation value.

A molding quality evaluation device according to the present disclosure is for evaluating molding quality of a molded product containing resin, and includes: a pair of electrodes placed to form a capacitor in the molded product; and a control unit that measures a capacitance value between the pair of electrodes and evaluates the molding quality of the molded product based on the measured capacitance value. The control unit is configured to perform the steps of: with a state of the molded product before molding being set as a reference state, acquiring a capacitance value of the molded product in the reference state as a reference value; with a state of the molded product being set as a molding state developed over time from the reference state and being a state of the molded product during molding, acquiring the capacitance value of the molded product in the molding state as a measurement value; deriving as a measurement evaluation value a value obtained by non-dimensionalizing the measurement value with the reference value; and evaluating the molding quality based on the measurement evaluation value.

A molded product inspection device according to the present disclosure is for inspecting the molding quality of the molded product based on an evaluation result of the above-described molding quality evaluation device. The molded product inspection device includes: a storage unit that stores therein a strength evaluation value satisfying a required strength of the molded product; and a control unit that determines whether the molded product satisfies the required strength, based on the strength evaluation value. The control unit is configured to perform the steps of: acquiring a sound evaluation value of the molded product whose molding quality has been previously evaluated to be sound; acquiring a defective evaluation value of the molded product whose molding quality has been previously evaluated to be defective; acquiring the measurement evaluation value derived in the molding quality evaluation device; and determining whether an inspection has been passed, based on the acquired measurement evaluation value and strength evaluation value. The strength evaluation value is set between the sound evaluation value and the defective evaluation value.

### Advantageous Effects of Invention

According to this disclosure, molding quality of a molded product can be suitably evaluated without affecting the molding quality.

### Brief Description of Drawings

FIG. 1 is a schematic configuration diagram illustrating a molded product inspection device according to a first embodiment.
FIG. 2 is an example of a flowchart related to a molding quality evaluation method according to the first embodiment.
FIG. 3 is a graph related to evaluation values that vary with time.
FIG. 4 is an example of a flowchart related to a molding quality evaluation method according to the first embodiment.
FIG. 5 is an example of a flowchart related to a molded product inspection method according to the first embodiment.
FIG. 6 is a graph of a defective evaluation value versus a sound evaluation value, varying with measurement frequency.
FIG. 7 is an explanatory diagram of the arrangement of electrodes of a molded product inspection device according to a second embodiment.
FIG. 8 is a schematic configuration diagram illustrating a molded product inspection device according to a third embodiment.
FIG. 9 is a schematic configuration diagram illustrating a molded product inspection device according to a fourth embodiment.
FIG. 10 is a schematic configuration diagram illustrating a molded product inspection device according to a fifth embodiment.
FIG. 11 is a schematic configuration diagram illustrating a molded product inspection device according to a seventh embodiment.

### Description of Embodiments

The following is a detailed description of embodiments according to the present disclosure based on the drawings. This disclosure is not limited by the embodiments. Also, the components in the following embodiments include those that are substitutable and easy for those skilled in the art, or those that are substantially the same. Furthermore, the components described below can be combined as appropriate, and when there is more than one embodiment, these embodiments can be combined.

### [First Embodiment]

A molding quality evaluation method, an inspection method of a molded product 1, and an inspection device 10 of the molded product 1 according to a first embodiment are methods and a device for measuring and evaluating the adhesive state of an adhesive portion 8 of the molded product 1. In the first embodiment, the inspection device 10 of the molded product 1 also serves as an evaluation device that evaluates molding quality.

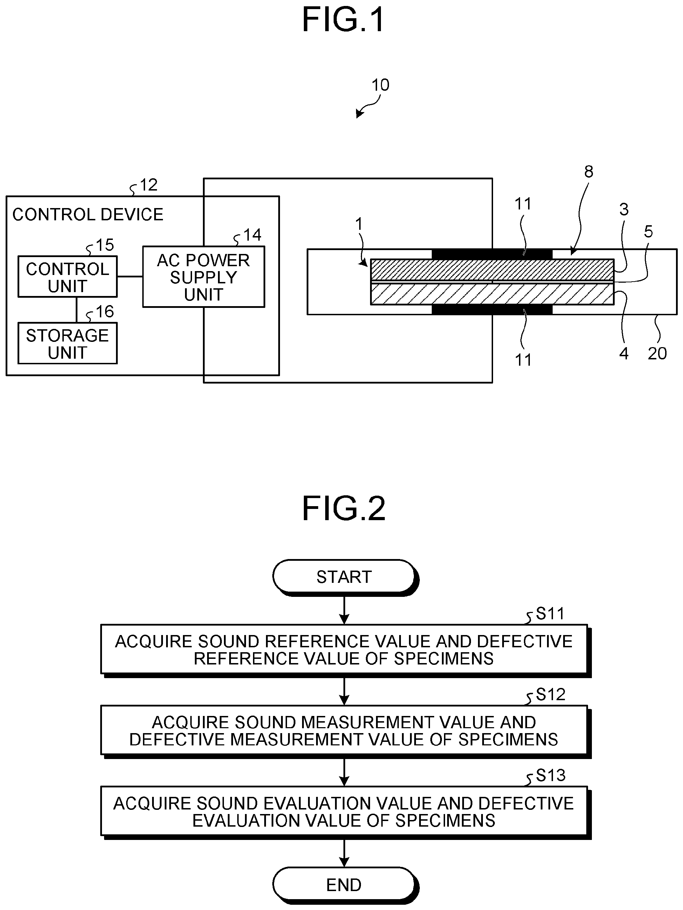

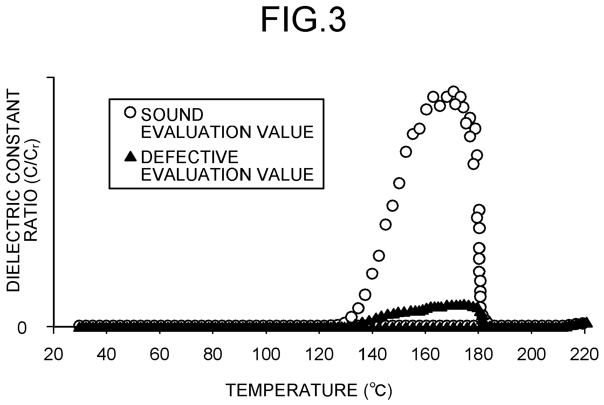

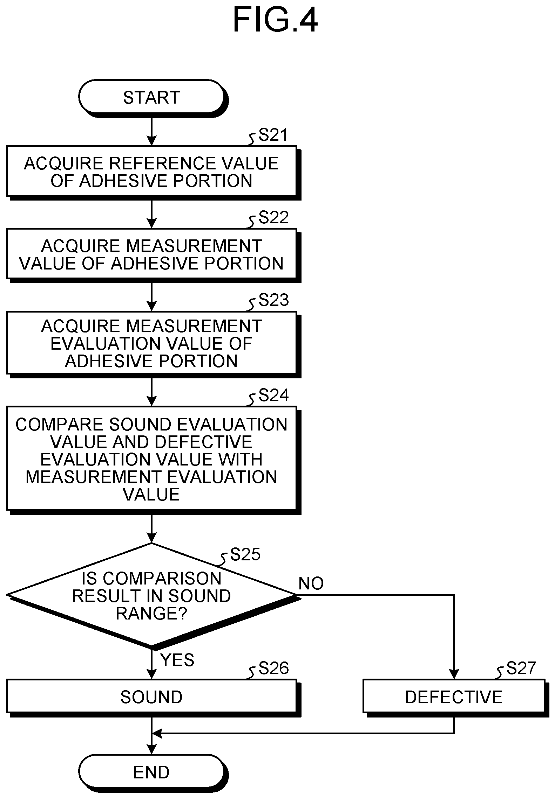

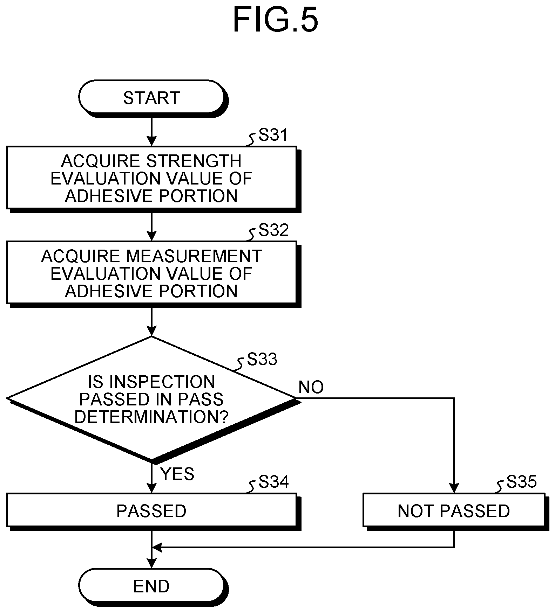

FIG. 1 is a schematic configuration diagram illustrating a molded product inspection device according to the first embodiment. FIGS. 2 and 4 are examples of flowcharts related to a molding quality evaluation method according to the first embodiment. FIG. 3 is a graph related to evaluation values that vary with time. FIG. 5 is an example of a flowchart related to a molded product inspection method according to the first embodiment. Before describing the molding quality evaluation method, the inspection method of the molded product 1, and the inspection device 10 of the molded product 1, the molded product 1 is described.

### (Molded Product)

The molded product 1 is an adhesive body formed by adhering a first adherend 3 and a second adherend 4 via an adhesive 5, and a portion at an adhesive portion between the first adherend 3 and the second adherend 4 is the adhesive portion 8. The molded product 1 may be, for example, a general part that is molded by adhesion, as well as a repair part that is repaired by adhesion. Although the first embodiment is described as being applied to the molded product 1 with the adhesive portions 8, it may also be a resin molded product without the adhesive portion 8.

Each of the first adherend 3 and the second adherend 4 is a composite material and is a laminate of composite layers containing carbon fiber and resin. The stacking direction of the laminate is an opposite direction in which the first adherend 3 and the second adherend 4 face each other. The first adherend 3 and the second adherend 4 are composite materials made of carbon fiber, but are not limited and any reinforcing fiber may be used. Thermosetting resins such as epoxy resins are used as resins. Furthermore, in the first embodiment, the first adherend 3 and the second adherend 4 are made of composite materials, but may also be made of resin materials or any combination of composite, metal, and resin materials. The adhesive 5 is a thermosetting resin that is thermally cured through a cross-linking reaction to bond the first adherend 3 and the second adherend 4.

Weak bonds, kissing bonds, porosity voids, or the like, which degrade adhesion quality, may occur in the adhesive portion 8. Therefore, the molding quality evaluation method and the molded product inspection method using the inspection device 10 evaluate whether the adhesive portion 8 of the molded product 1 is sound. That is, in the first embodiment, adhesion quality is evaluated as molding quality, and in order to evaluate the adhesion quality, the presence or absence of adhesion defects is evaluated. In the first embodiment, the molded product is inspected to determine whether an adhesive strength meets a required strength.

### (Molded Product Inspection Device)

As illustrated in FIG. 1, the inspection device 10 includes a pair of electrodes 11 and a control device 12.

The molded product 1 before molding is interposed between the pair of electrodes 11 from both sides in the facing direction thereof, so that the pair of electrodes 11 form a capacitor. Therefore, the molded product 1 is placed between the pair of electrodes 11 as a dielectric. Each electrode 11 is formed of a metal thin film, for example, and is placed over the entire surface of the adhesive portion 8 of the molded product 1. The molded product 1 before molding with the pair of electrodes 11 placed is accommodated inside a bagging film 20. The inside of the bagging film 20 is vacuumed, and the molded product 1 is thermally cured by heating while applying pressure with atmospheric pressure to the molded product 1.

The control device 12 includes an AC power supply unit 14, a control unit 15, and a storage unit 16. The AC power supply unit 14 applies an AC signal between the pair of electrodes 11. The AC signal is an AC current or an AC voltage. The AC power supply unit 14 is, for example, an LCR meter. The AC power supply unit 14 can apply the AC signal between the pair of electrodes 11 by varying the frequency of the AC signal. The AC power supply unit 14 is connected to the control unit 15, and the control unit 15 controls the AC signal to be applied so that the frequency of the AC signal is a predetermined frequency. The AC power supply unit 14 may superimpose a DC signal on the AC signal and apply the superimposed signal between the pair of electrodes 11. The AC power supply unit 14 can reduce an adverse effect of a signal-to-noise ratio (S/N) by superimposing the DC signal, thereby enabling accurate measurement of a capacitance value of the capacitor formed by the pair of electrodes 11.

The control unit 15, for example, includes an integrated circuit such as a central processing unit (CPU). The control unit 15 performs an evaluation process of evaluating the adhesive state of the adhesive portion 8. The control unit 15 varies the frequency of the AC signal by controlling the AC power supply unit 14 in the evaluation process.

The storage unit 16 is any storage device such as a semiconductor storage device and a magnetic storage device. The storage unit 16 stores therein various computer programs and various data. The storage unit 16 stores therein, as various data, a sound evaluation value of a molded product whose adhesive portion 8 has been previously evaluated to be sound and a defective evaluation value being an evaluation value of the molded product whose adhesive portion 8 has been previously evaluated to be defective. The storage unit 16 also stores therein, as various data, previously acquired strength evaluation values satisfying a required adhesive strength at the adhesive portion 8 of the molded product 1.

### (Molding Quality Evaluation Method)

An example of the molding quality evaluation method performed in the inspection device 10 of the molded product 1 is described below with reference to FIGS. 2 to 4.

The molding quality evaluation method acquires in advance a sound evaluation value being an evaluation value of a specimen with a sound adhesive portion 8, and a defective evaluation value being an evaluation value of a specimen with a defective adhesive portion 8. Subsequently, the method acquires a measurement evaluation value being the evaluation value of the adhesive portion 8 of the molded product 1 to be evaluated, and evaluates the adhesive state of the adhesive portion 8 based on the sound evaluation value, the defective evaluation value, and the measurement evaluation value. At this time, the shapes of the sound and defective adhesive portions 8 of the specimens and the shape of the adhesive portion 8 of the molded product 1 to be measured are identical. The specimen with the sound adhesive portion 8 is one in which no adhesion defects such as week bonds, kissing bonds, and porosity voids have occurred. The specimen with the defective adhesive portion 8 is a specimen that simulates adhesion defects, for example, a specimen with a release agent on the entire adhesive surface of the specimen.

First, the process of acquiring the sound evaluation value and the defective evaluation value is described with reference to FIG. 2. As illustrated in FIG. 2, in the molding quality evaluation method, the control unit 15 first performs step S11 of measuring a capacitance value by using the specimen instead of the molded product 1 and acquiring a sound reference value and a defective reference value. The reference value is the capacitance value of the adhesive portion 8 in a reference state that serves as a reference for the molded product or the specimen. The sound reference value is the capacitance value of the adhesive portion 8 in the reference state of the specimen with the sound adhesive portion 8. The defective reference value is the capacitance value of the adhesive portion 8 in the reference state of the specimen with the defective adhesive portion 8. The reference state is the state of the molded product or the specimen before molding, that is, before the cross-linking reaction of the adhesive 5 occurs or the initial state of the cross-linking reaction of the adhesive 5, where the adhesive 5 is in an uncured state. Specifically, the reference state is managed as a reference temperature serving as a reference in the adhesive portion 8, and the reference temperature is an environmental temperature around the adhesive portion 8 and is a room temperature. The room temperature ranges from 1°C to 35°C and the reference temperature is, for example, 30°C.

The measurement frequency of the AC signal applied at the time of measuring the capacitance value is in a range of 10 Hz or more to less than 10 kHz, and the capacitance value is measured with the frequency fixed at a predetermined frequency. That is, at step S11, the environmental temperature around the specimen before molding is the room temperature, and by using this temperature as the reference temperature, the control unit 15 applies an AC signal with the predetermined frequency to the pair of electrodes 11 to acquire the capacitance value of the adhesive portion 8 of the specimen as the sound reference value or the defective reference value.

Subsequently, in the molding quality evaluation method, the control unit 15 performs step S12 of acquiring the sound measurement value being the measurement value of the specimen with the sound adhesive portion 8, and the defective measurement value being the measurement value of the specimen with the defective adhesive portion 8. The measurement value is the capacitance value of the adhesive portion 8 in the molding state of the molded product or the specimen that has been developed over time from the reference state. The sound measurement value is the capacitance value of the adhesive portion 8 of the specimen with the adhesive portion 8 that is sound in the molding state that has been developed over time from the reference state. The defective measurement value is the capacitance value of the adhesive portion 8 of the specimen with the adhesive portion 8 that is defective in the molding state that has been developed over time from the reference state. The molding state is the state in which the molded product 1 or the specimen is being molded, that is, the state in which the cross-linking reaction of the adhesive 5 is underway and the curing of the adhesive 5 is in progress. Specifically, the molding state is managed as the molding temperature during molding in the adhesive portion 8, where the molding temperature is, for example, the glass transition temperature of the adhesive 5. That is, the molding temperature is higher than the reference temperature. In the first embodiment, since a thermosetting resin was applied as the adhesive 5, the reference state and the molding state of the molded product 1 were managed by temperature; however, the present disclosure is not limited thereto. When a room temperature curing resin is applied as the adhesive 5, the reference state and molding state of the molded product 1 or the specimen may be managed by time. In this case, the molding state of the molded product 1 or the specimen is more time-lapsed than the reference state of the molded product 1 or the specimen.

At step S12, the sound measurement value and the defective measurement value are acquired at predetermined temperature intervals. The measurement frequency of the AC signal applied at the time of measuring the capacitance value is the same frequency as in step S11. That is, at step S12, the environmental temperature around the specimen before molding is the molding temperature, and the control unit 15 applies an AC signal with a predetermined frequency to the pair of electrodes 11 to acquire the capacitance values of the adhesive portion 8 as the sound measurement value and the defective measurement value.

Subsequently, in the molding quality evaluation method, the control unit 15 performs step S13 of deriving the sound measurement value referenced by the sound reference value as a sound evaluation value and similarly, deriving the defective measurement value referenced by the defective reference value as a defective evaluation value. The reference is the non-dimensionalization of a measurement value with a reference value. Here, a capacitance value C is given as the product of a dielectric constant ε and a shape factor f(r), the formula "C = ε × f(r)". The shape factor f(r) is given, for example, as the formula "f(r) = S/d", where a parallel plate area S is divided by a distance d between parallel plates when the pair of electrodes 11 serve as a parallel plate capacitor. It is assumed that a reference value is Cᵣ, a dielectric constant at the reference value is εᵣ, a measurement value is C, and a dielectric constant at the measurement value is ε. An evaluation value being a value (C/Cᵣ) obtained by referencing the measurement value C with the reference value Cᵣ is "C/Cᵣ = ε/εᵣ" due to the cancellation of the shape factor f(r). That is, at step S13, the sound evaluation value and the defective evaluation value are derived as "ε/εᵣ (dielectric constant ratio)" in each specimen as the evaluation value.

FIG. 3 is a graph of the dielectric constant ratio that varies with time development (including temperature and time), where a horizontal axis denotes time and a vertical axis denotes the measurement value C referenced by the reference value Cᵣ, that is, the dielectric constant ratio. In FIG. 3, the measurement frequency is 100 Hz. The triangular marks in FIG. 3 are defective evaluation values that vary with temperature, and the circular marks are sound evaluation values that vary with temperature. As illustrated in FIG. 3, during a temperature increase process from 120°C to 180°C, the dielectric constant ratio being the sound evaluation value changes significantly compared to the dielectric constant ratio being the defective evaluation value. In particular, the peak value of the dielectric constant ratio being the sound evaluation value is larger than the peak value of the dielectric constant ratio being the defective evaluation value. Subsequently, at step S13, the control unit 15 acquires the sound evaluation value and the defective evaluation value that vary as illustrated in FIG. 3.

Strength evaluation values are set based on various evaluation values including sound evaluation values and defective evaluation values according to the adhesive state of the adhesive portion 8, and an adhesive strength associated with the evaluation values. The strength evaluation value is set as a threshold value, with the evaluation value corresponding to a required adhesive strength that is required. For example, in FIG. 3, the strength evaluation value is set between the sound evaluation value and the defective evaluation value.

The process of acquiring measurement evaluation values and evaluating the adhesive state of the adhesive portion 8 of the molded product 1 to be measured is described below with reference to FIG. 4. As illustrated in FIG. 4, in the molding quality evaluation method, the control unit 15 first performs step S21 of acquiring the reference value of the adhesive portion 8 to be measured. A reference state at step S21 is the same as in step S11. That is, at step S21, the environmental temperature around the adhesive portion 8 is the room temperature, and by using this temperature as the reference temperature, the control unit 15 applies an AC signal with a predetermined frequency to the pair of electrodes 11 to acquire the capacitance value of the adhesive portion 8 as the reference value.

Subsequently, in the molding quality evaluation method, the control unit 15 performs step S22 of acquiring the measurement value of the adhesive portion 8 to be measured. A molding state at step S22 is the same as in step S12. That is, at step S22, the environmental temperature around the adhesive portion 8 is the molding temperature, and the control unit 15 applies an AC signal with a predetermined frequency to the pair of electrodes 11 to acquire the capacitance value of the adhesive portion 8 as the measurement value. At step S22, the control unit 15 acquires the measurement value at predetermined temperature intervals.

Subsequently, the molding quality evaluation method, the control unit 15 performs step S23 of deriving the measurement value referenced by the reference value as the measurement evaluation value. That is, at step S23, "ε/εᵣ (dielectric constant ratio)" is derived as the measurement evaluation value. The measurement evaluation value is acquired as a dielectric constant ratio that varies with temperature, for example, like the sound evaluation value and the defective evaluation value illustrated in FIG. 3.

Subsequently, in the molding quality evaluation method, the control unit 15 performs steps S24 to S27 of evaluating the adhesive portion 8 based on the previously acquired sound and defective evaluation values and the measurement evaluation value. Specifically, the control unit 15 compares the sound evaluation value and the defective evaluation value with the measurement evaluation value (step S24). The control unit 15 performs step S25 of determining whether the comparison result is in a sound range. At step S25, for example, the control unit 15 may evaluate, as the soundness of the adhesive portion 8, the ratio of the absolute value of a difference between the measurement evaluation value and the sound evaluation value to the absolute value of a difference between the measurement evaluation value and the defective evaluation value in the range between the sound evaluation value and the defective evaluation value, and determine whether the evaluated soundness is within a specified soundness range specified in advance. At step S25, for example, the above strength evaluation value may be used as a threshold value for evaluating the soundness of the adhesive portion 8. Furthermore, at step S25, the control unit 15 may compare, for example, the time-varying trajectory of the measurement evaluation value with the time-varying trajectory of the sound and defective evaluation values. That is, any evaluation method may be used to evaluate the soundness of the adhesive portion 8, as long as the evaluation is based on the previously acquired sound and defective evaluation values and the measurement evaluation value.

When it is determined at step S25 that the comparison result is in the sound range (Yes at step S25), the control unit 15 evaluates that the adhesive portion 8 is sound (step S26) and completes the process of evaluating the adhesive state of the adhesive portion 8. On the other hand, when it is determined at step S25 that the comparison result is not in the sound range (No at step S25), the control unit 15 evaluates that the adhesive portion 8 is defective, that is, that the adhesive portion 8 has adhesion defects such as week bonds, kissing bonds, or porosity voids (step S27), and completes the process of evaluating the adhesive state of the adhesive portion 8. For accurate evaluation of the adhesion defects, a specimen with week bonds, kissing bonds, or porosity voids is preferably used as the specimen used to acquire the defective evaluation value.

In the first embodiment, the soundness of the adhesive portion 8 is evaluated based on the previously acquired sound evaluation value and defective evaluation value and the measurement evaluation value, but the soundness evaluation may be performed based on the measurement evaluation value. For example, a threshold value for determining soundness may be set, and the soundness of the adhesive portion 8 may be evaluated according to whether the measurement evaluation value exceeds the threshold value.

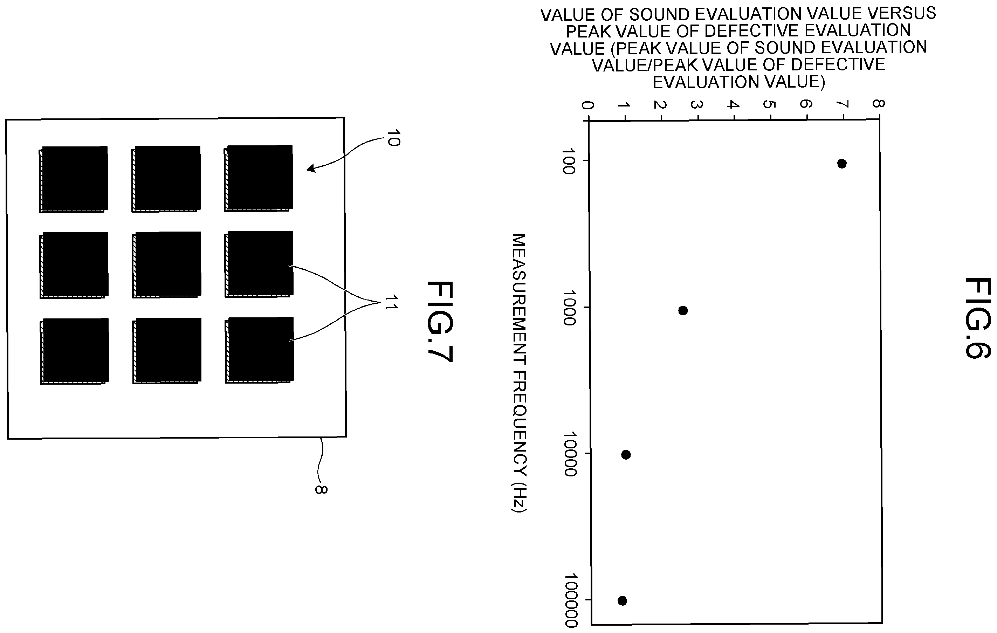

The measurement frequency is described below with reference to FIG. 6. FIG. 6 is a graph of the defective evaluation value versus the sound evaluation value, varying with measurement frequency, where a horizontal axis denotes the measurement frequency (Hz) and a vertical axis denotes the peak value of the sound evaluation value versus the peak value of the defective evaluation value. Specifically, the vertical axis denotes (peak value of sound evaluation value/peak value of defective evaluation value). As illustrated in FIG. 6, when the measurement frequency is 100 Hz (the case of FIG. 3), peak value of the sound evaluation value/peak value of the defective evaluation value is about 7, and the peak value of the sound evaluation value and the peak value of the defective evaluation value are significantly different values. When the measurement frequency is 1 kHz, peak value of the sound evaluation value/peak value of the defective evaluation value is about 2 to 3, and the peak value of the sound evaluation value and the peak value of the defective evaluation value are significantly different values. When the measurement frequency is 10 kHz and 100 kHz, peak value of the sound evaluation value/peak value of the defective evaluation value is about 1, and the peak value of the sound evaluation value and the peak value of the defective evaluation value are almost the same value. Therefore, when the measurement frequency is smaller than 10 kHz, the difference between the peak value of the sound evaluation value and the peak value of the defective evaluation value is increased, allowing a detailed evaluation of soundness based on the measurement evaluation value.

### (Molded Product Inspection Method)

An example of an inspection method of the molded product 1 performed by the inspection device 10 of the molded product 1 is described below with reference to FIG. 5. The inspection method of the molded product 1 is a method for inspecting the adhesive strength of the adhesive portion 8. With the inspection method of the molded product 1, whether a required adhesive strength is satisfied is inspected based on strength evaluation values acquired in advance and the measurement evaluation values acquired in the molding quality evaluation method.

As illustrated in FIG. 5, in the inspection method of the molded product 1, the control unit 15 first performs step S31 of acquiring the strength evaluation value of the adhesive portion 8 to be measured. At step S31, the control unit 15 acquires the strength evaluation values stored in the storage unit 16.

Subsequently, in the inspection method of the molded product 1, the control unit 15 performs step S32 of acquiring the measurement evaluation value of the adhesive portion 8. At step S32, steps S21 to S23 in the process of FIG. 4 are performed. Therefore, a description of step S32 is omitted.

Subsequently, in the inspection method of the molded product 1, the control unit 15 performs step S33 of determining whether the inspection is passed, based on the acquired measurement evaluation values and strength evaluation values. At step S33, for example, the control unit 15 determines whether the inspection is passed, based on whether the measurement evaluation value is on the sound side or the defective side with respect to the strength evaluation value. When it is determined at step S33 that the measurement evaluation value is on the sound side with respect to the strength evaluation value (Yes at step S33), the control unit 15 determines that the adhesive portion 8 satisfies the required adhesive strength and that the inspection is passed (step S34), and completes the process related to the inspection method of the molded product 1. On the other hand, when it is determined at step S33 that the measurement evaluation value is not on the sound side with respect to the strength evaluation value (No at step S33), the control unit 15 determines that the adhesive portion 8 does not satisfy the required adhesive strength and that the inspection is not passed (step S35), and completes the process related to the inspection method of the molded product 1.

In the first embodiment, the first adherend 3 and the second adherend 4 are made of composite materials, but may also be made of metal materials. In this case, the first adherend 3 and the second adherend 4 serve as electrodes, and an adhesive layer between the first adherend 3 and the second adherend 4 serves as a dielectric.

### [Second Embodiment]

A second embodiment is described below with reference to FIG. 7. FIG. 7 is an explanatory diagram of the arrangement of electrodes of a molded product inspection device according to the second embodiment. In the second embodiment, in order to avoid duplicate descriptions, parts different from those of the first embodiment are described, and parts having the same configuration as in the first embodiment are described with the same symbols.

In an inspection device 10 of the second embodiment, each of the pair of electrodes 11 is placed in plurality in the adhesive portion 8. Specifically, the pair of electrodes 11 are smaller than in the first embodiment, and are placed on a portion of the surface of the adhesive portion 8, and the pair of electrodes 11 are placed in plurality to be distributed in the adhesive portion 8. The plurality of pairs of electrodes 11 are placed in a grid pattern, for example.

When the molding quality evaluation method is performed using the inspection device 10 described above, the adhesive state can be acquired as a distribution of measurement evaluation values in the adhesive portion 8. Therefore, the adhesive state of the adhesive portion 8 can be acquired in detail.

The molding quality evaluation results evaluated by the inspection device 10 may be used in the molding process of the molded product 1. Specifically, the molding process of the molded product 1 may adjust the molding conditions of the molded product 1 based on the evaluation results to improve the molding quality of the molded product 1.

### [Third Embodiment]

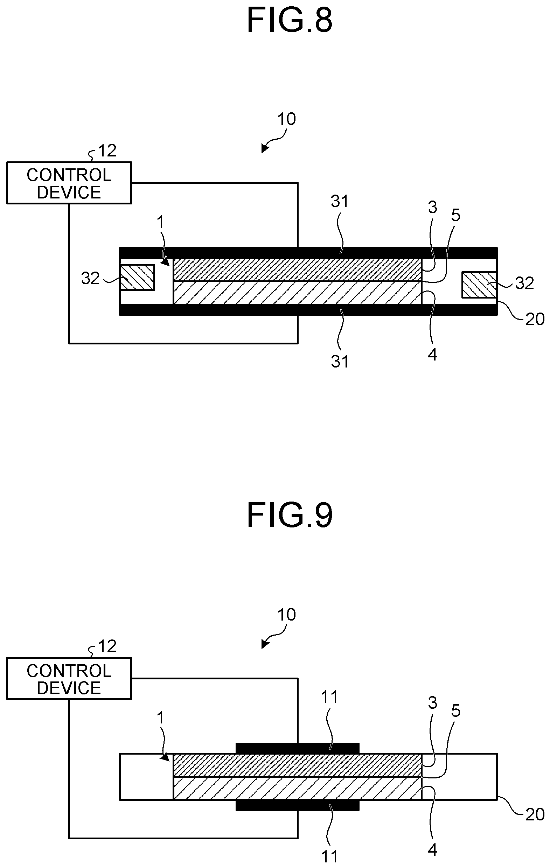

A third embodiment is described below with reference to FIG. 8. FIG. 8 is a schematic configuration diagram illustrating a molded product inspection device according to the third embodiment. In the third embodiment as well, in order to avoid duplicate descriptions, parts different from those of the first and second embodiments are described, and parts having the same configuration as in the first and second embodiments are described with the same symbols.

In an inspection device 10 of the third embodiment, the pair of electrodes 11 are formed as a pair of electrode layers 31 formed inside the bagging film 20. The inspection device 10 further includes an insulating spacer 32 that maintains an insulating state between the pair of electrode layers 31.

The pair of electrode layers 31 are formed at opposite positions while interposing the molded product 1 before molding that is accommodated inside the bagging film 20 therebetween. The electrode layer 31 may be formed by vapor deposition. The insulating spacer 32 is provided between the pair of electrode layers 31 and is placed on both sides of the molded product 1 that is accommodated inside the bagging film 20. As in the second embodiment, the pair of electrode layers 31 may be provided in plurality.

### [Fourth Embodiment]

A fourth embodiment is described below with reference to FIG. 9. FIG. 9 is a schematic configuration diagram illustrating a molded product inspection device according to the fourth embodiment. In the fourth embodiment as well, in order to avoid duplicate descriptions, parts different from those of the first to third embodiments are described, and parts having the same configuration as in the first to third embodiments are described with the same symbols.

In an inspection device 10 of the fourth embodiment, the pair of electrodes 11 are located at positions different from the positions of the pair of electrodes 11 in the first embodiment. In the first embodiment, the pair of electrodes 11 are placed at the adhesive portion 8 of the molded product 1 inside the bagging film 20. In the fourth embodiment, the pair of electrodes 11 are placed at the adhesive portion 8 of the molded product 1 outside the bagging film 20. That is, in the fourth embodiment, the pair of electrodes 11 are provided outside the bagging film 20 at positions facing the adhesive portion 8 of the molded product 1 after the molded product 1 is accommodated inside the bagging film 20 and vacuum suctioned. As in the second embodiment, the pair of electrodes 11 may be provided in plurality.

### [Fifth Embodiment]

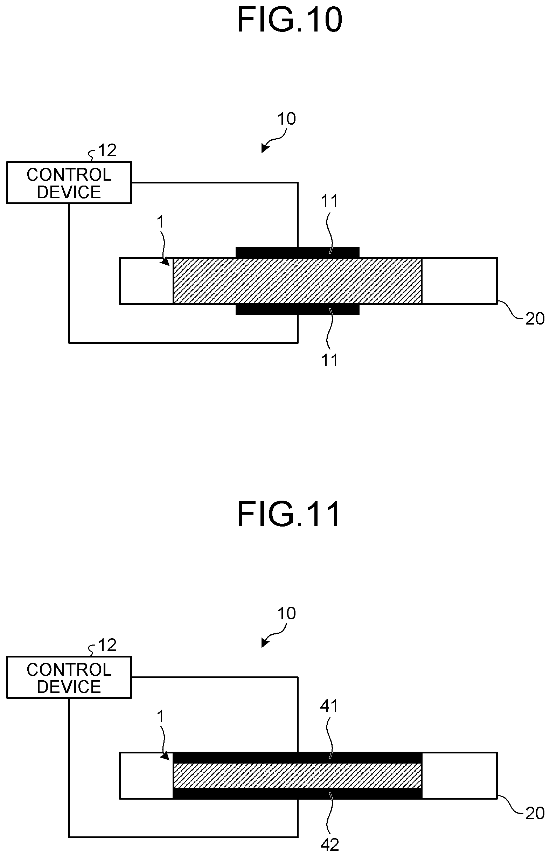

A fifth embodiment is described below with reference to FIG. 10. FIG. 10 is a schematic configuration diagram illustrating a molded product inspection device according to the fifth embodiment. In the fifth embodiment as well, in order to avoid duplicate descriptions, parts different from those of the first to fourth embodiments are described, and parts having the same configuration as in the first to fourth embodiments are described with the same symbols.

In the fifth embodiment, a molded product 1 to be measured is not the adhesive body of the first to fourth embodiments, but a resin molded product without the adhesive portion 8. That is, in the fifth embodiment, the molding quality of the resin molded product is evaluated using the molding quality evaluation method. Since a measurement target is the resin molded product, a sound evaluation value, a defective evaluation value, and a strength evaluation value of a molded product to be stored in the storage unit 16 are acquired in advance using a specimen that imitates the resin molded product. The sound evaluation value, the defective evaluation value, and the strength evaluation value are acquired by the same method as in the first embodiment. In addition to thermosetting resins, thermoplastic resins may also be applied as resins used for the resin molded product.

### [Sixth Embodiment]

A sixth embodiment is described below. In the sixth embodiment as well, in order to avoid duplicate descriptions, parts different from those of the first to fifth embodiments are described, and parts having the same configuration as in the first to fifth embodiments are described with the same symbols.

In the sixth embodiment, a molded product 1 to be measured is an adhesive body similar to the first embodiment, and a room temperature curable resin is applied as a resin to be used instead of a thermosetting resin. When the room temperature curing resin is applied, reference and molding states are managed by time. The inspection device 10, the molding quality evaluation method, and the inspection method of the molded product 1 are similar to those of the first embodiment, except that the reference state and the molding state are managed by time.

### [Seventh Embodiment]

A seventh embodiment is described below with reference to FIG. 11. FIG. 11 is a schematic configuration diagram illustrating a molded product inspection device according to the seventh embodiment. In the seventh embodiment as well, in order to avoid duplicate descriptions, parts different from those of the first to sixth embodiments are described, and parts having the same configuration as in the first to sixth embodiments are described with the same symbols.

In an inspection device 10 of the seventh embodiment, the pair of electrodes 11 include at least one of a metal mesh 41 provided inside the molded product 1 and a metal jig 42 used during the molding of the molded product 1. Specifically, in the seventh embodiment, one of the pair of electrodes 11 is the metal mesh 41 and the other electrode 11 is the metal jig 42. Both of the pair of electrodes 11 may be the metal mesh 41 or the metal jig 42, without any limitation. The metal mesh 41, for example, is a lightning-resistant member that is integrally provided with the molded product 1 in order to protect a molded product from being damaged by lightning currents during lightning strike. The metal jig 42 is, for example, a mold for forming the molded product 1. That is, in the seventh embodiment, a part of the molded product or a member used in the molding process is utilized as the pair of electrodes 11, without separately preparing the pair of electrodes 11.

In the first to seventh embodiments, the inspection device 10 of the molded product 1 also serves as an evaluation device for evaluating molding quality; however, the evaluation device for evaluating molding quality and the inspection device for inspecting the molded product 1 may be configured as separate devices.

As described above, the evaluation method of the molding quality of a composite material, the inspection method of the molded product 1, the molding quality evaluation device, and the inspection device 10 of the molded product 1 described in the first to seventh embodiments are understood, for example, as follows.

A molding quality evaluation method according to a first aspect is a molding quality evaluation method for evaluating the molding quality of the molded product 1 containing resin, and includes step S21 of setting the state of the molded product 1 before molding as a reference state and acquiring the capacitance value of the molded product 1 in the reference state as a reference value, step S22 of setting the state of the molded product 1 as a molding state developed over time from the reference state and being the state of the molded product 1 during molding and acquiring the capacitance value of the molded product 1 in the molding state as a measurement value, step S23 of deriving, as a measurement evaluation value, a value obtained by non-dimensionalizing the measurement value with the reference value, and steps S24 to S27 of evaluating the molding quality based on the measurement evaluation value.

According to this configuration, the molding quality can be evaluated during the molding of the molded product 1, so that heating or the like after molding need not be performed and the molding quality of the molded product 1 can be prevented from being affected. In addition, by referencing the measurement value with the reference value, a measurement evaluation value that cancels the influence of shape can be obtained. Therefore, the molding quality of the molded product 1 can be suitably evaluated using the measurement evaluation value.

As a second aspect, the molding quality evaluation method according to the first aspect further includes step S13 of acquiring a sound evaluation value of the molded product whose molding quality has been previously evaluated to be sound, and step S13 of acquiring a defective evaluation value of the molded product whose molding quality has been previously evaluated to be defective. At steps S24 to S27 of evaluating the molding quality, the molding quality is evaluated based on the sound evaluation value, the defective evaluation value, and the measurement evaluation value.

According to this configuration, the evaluation of the molding quality based on the measurement evaluation value can be performed with high accuracy by using the sound evaluation value and the defective evaluation value as indicators.

As a third aspect, in the molding quality evaluation method according to the first or second aspect, the resin is a thermosetting resin, the reference state is a state before a cross-linking reaction of the thermosetting resin or a state at the start of cross-linking of the thermosetting resin, and the molding state is a state in which a predetermined time has elapsed after the start of the cross-linking of the thermosetting resin.

According to this configuration, when the thermosetting resin is used as the resin, the evaluation of the molding quality due to the cross-linking reaction can be performed.

As a fourth aspect, in the molding quality evaluation method according to the third aspect, the reference state is a state of temperature lower than the glass transition temperature of the thermosetting resin, and the molding state is a state of temperature equal to or higher than the glass transition temperature of the thermosetting resin.

According to this configuration, the reference state and the molding state can be managed by temperatures including the glass transition temperature.

As a fifth aspect, in the molding quality evaluation method according to the third or fourth aspect, the capacitance value is acquired by applying an AC signal with a predetermined measurement frequency, and the measurement frequency is in a range of 10 Hz or more to less than 10 kHz.

According to this configuration, the measurement frequency can be set to a frequency suitable for the evaluation of the molding quality when the thermosetting resin is used, thereby allowing the evaluation of the molding quality to be performed with high accuracy.

As a sixth aspect, in the molding quality evaluation method according to any one of the first to fifth aspects, the molded product is an adhesive body to which the first adherend 3 and the second adherend 4 adhere via the adhesive 5, and the molding quality is the adhesion quality of the adhesive 5.

According to this configuration, the adhesion quality of the adhesive body can be suitably evaluated.

As a seventh aspect, in the molding quality evaluation method according to the sixth aspect, the step of evaluating the molding quality evaluates the occurrence of adhesion defects, including week bonds, kissing bonds, and porosity voids, as the adhesion quality.

According to this configuration, the adhesion defects of week bonds, kissing bonds, and porosity voids can be evaluated as the adhesion quality of the adhesive body.

As an eighth aspect, in the molding quality evaluation method according to any one of the first to seventh aspects, at step S21 of acquiring the reference value and at step S22 of acquiring the measurement value, a capacitance value is measured at a plurality of locations in the molded product 1, at step S23 of deriving the measurement evaluation value, the measurement evaluation value is derived at the plurality of locations of the molded product 1, and at steps S24 to S27 of evaluating the molding quality, the distribution of the molding quality in the molded product 1 is evaluated based on a plurality of the measurement evaluation values.

According to this configuration, the distribution of the molding quality can be acquired as an evaluation result, thereby allowing detailed evaluation of the molding quality to be performed.

A molded product inspection method according to a ninth aspect is an inspection method of the molded product 1 for inspecting the molding quality of the molded product 1 based on the evaluation results of the above molding quality evaluation method, a strength evaluation value satisfying the required strength of the molded product 1 being acquired in advance, the molded product inspection method including step S13 of acquiring a sound evaluation value of the molded product whose molding quality has been previously evaluated to be sound, step S13 of acquiring a defective evaluation value of the molded product whose molding quality has been previously evaluated to be defective, step S32 of acquiring the measurement evaluation value derived in the molding quality evaluation method, and step S33 of determining whether the inspection has been passed, based on the acquired measurement evaluation value and strength evaluation value. The strength evaluation value is set between the sound evaluation value and the defective evaluation value.

According to this configuration, whether the molding quality satisfies the required strength can be inspected.

A molding quality evaluation device according to a tenth aspect is a molding quality evaluation device (inspection device 10) for evaluating the molding quality of the molded product 1 containing resin, and includes the pair of electrodes 11 placed to form a capacitor in the molded product 1 and the control unit 15 that measures a capacitance value between the pair of electrodes 11 and evaluates the molding quality of the molded product 1 based on the measured capacitance value. The control unit 15 performs step S21 of setting the state of the molded product 1 before molding as a reference state and acquiring the capacitance value of the molded product 1 in the reference state as a reference value, step S22 of setting the state of the molded product 1 as a molding state developed over time from the reference state and being the state of the molded product 1 during molding and acquiring the capacitance value of the molded product 1 in the molding state as a measurement value, step S23 of deriving, as a measurement evaluation value, a value obtained by non-dimensionalizing the measurement value with the reference value, and steps S24 to S27 of evaluating the molding quality based on the measurement evaluation value.

According to this configuration, the molding quality can be evaluated during the molding of the molded product 1, so that heating or the like after molding need not be performed and the molding quality of the molded product 1 can be prevented from being affected. In addition, by referencing the measurement value with the reference value, a measurement evaluation value that cancels the influence of shape can be obtained. Therefore, the molding quality of the molded product 1 can be suitably evaluated using the measurement evaluation value.

As an eleventh aspect, in the molding quality evaluation device according to the tenth aspect, the pair of electrodes 11 are metal thin films.

According to this configuration, the electrodes 11, which are metal thin films, can be easily installed on the molded product 1.

As a twelfth aspect, in the molding quality evaluation device according to the tenth or eleventh aspect, the pair of electrodes 11 are provided at a plurality of locations on the molded product 1.

According to this configuration, the distribution of the molding quality can be acquired as an evaluation result, thereby allowing detailed evaluation of the molding quality to be performed.

As a thirteenth aspect, the molding quality evaluation device according to the tenth aspect further includes the bagging film 20 that accommodates the molded product 1 inside and the insulating spacer 32 that maintains an insulating state between the pair of electrodes 11, and the pair of electrodes 11 are the pair of electrode layers 31 formed inside the bagging film 20.

According to this configuration, the pair of electrodes 11 can be placed inside the bagging film 20 by accommodating the molded product 1 before molding, thereby improving workability.

As a fourteenth aspect, the molding quality evaluation device according to the tenth aspect further includes the bagging film 20 that accommodates the molded product 1 inside, and the pair of electrodes 11 are provided outside the bagging film 20.

According to this configuration, the workability can be improved because the pair of electrodes 11 can be placed inside the bagging film 20 after the molded product 1 before molding is accommodated inside.

As a fifteenth aspect, in the molding quality evaluation device according to the tenth aspect, the pair of electrodes 11 include at least one of the metal mesh 41 provided inside the molded product 1 and the metal jig 42 used during the molding of the molded product 1.

According to this configuration, existing members can be utilized as the pair of electrodes 11, which improves workability because there is no work to place the pair of electrodes 11.

A molding quality evaluation device according to a sixteenth aspect is the inspection device 10 of the molded product 1 for inspecting the molding quality of the molded product 1 based on the evaluation results of the above molding quality evaluation device, and includes the storage unit 16 that stores therein a strength evaluation value satisfying a required strength of the molded product 1 and the control unit 15 that determines whether the molded product 1 satisfies the required strength, based on the strength evaluation value. The control unit 15 performs step S13 of acquiring a sound evaluation value of the molded product whose molding quality has been previously evaluated to be sound, step S13 of acquiring a defective evaluation value of the molded product whose molding quality has been previously evaluated to be defective, step S32 of acquiring the measurement evaluation value derived in the molding quality evaluation device, step S33 of determining whether the inspection has been passed, based on the acquired measurement evaluation value and strength evaluation value, and step S34 of determining that the inspection has been passed when the measurement evaluation value is equal to or less than the strength evaluation value. The strength evaluation value is set between the sound evaluation value and the defective evaluation value.

According to this configuration, whether the molding quality satisfies the required strength can be inspected.

### Reference Signs List

- 1: Molded product
- 3: First adherend
- 4: Second adherend
- 5: Adhesive
- 8: Adhesive portion
- 10: Inspection device
- 11: Electrode
- 12: Control device
- 14: AC power supply unit
- 15: Control unit
- 16: Storage unit
- 20: Bagging film
- 31: Electrode layer
- 32: Insulating spacer
- 41: Metal mesh
- 42: Metal jig

## Claims

1. A molding quality evaluation method for evaluating molding quality of a molded product containing resin, the molding quality evaluation method comprising the steps of:
with a state of the molded product before molding being set as a reference state, acquiring as a reference value a capacitance value of the molded product in the reference state;
with a state of the molded product during molding being set as a molding state developed over time from the reference state, acquiring as a measurement value the capacitance value of the molded product in the molding state;
deriving as a measurement evaluation value a value obtained by non-dimensionalizing the measurement value with the reference value; and
evaluating the molding quality based on the measurement evaluation value.

2. The molding quality evaluation method according to claim 1, further comprising the steps of:
acquiring a sound evaluation value of the molded product whose molding quality has been previously evaluated to be sound; and
acquiring a defective evaluation value of the molded product whose molding quality has been previously evaluated to be defective, wherein
the step of evaluating the molding quality includes evaluating the molding quality based on the sound evaluation value, the defective evaluation value, and the measurement evaluation value.

3. The molding quality evaluation method according to claim 1, wherein
the resin is a thermosetting resin,
the reference state is a state before a cross-linking reaction of the thermosetting resin or a state at a start of cross-linking of the thermosetting resin, and
the molding state is a state in which a predetermined time has elapsed after the start of the cross-linking of the thermosetting resin.

4. The molding quality evaluation method according to claim 3, wherein
the reference state is a state of temperature lower than glass transition temperature of the thermosetting resin, and
the molding state is a state of temperature equal to or higher than the glass transition temperature of the thermosetting resin.

5. The molding quality evaluation method according to claim 3, wherein
the capacitance value is acquired by applying an AC signal with a predetermined measurement frequency, and
the measurement frequency is in a range of 10 Hz or more to less than 10 kHz.

6. The molding quality evaluation method according to claim 1, wherein
the molded product is an adhesive body to which a first adherend and a second adherend adhere via an adhesive, and
the molding quality is an adhesive quality of the adhesive.

7. The molding quality evaluation method according to claim 6, wherein the step of evaluating the molding quality includes evaluating as the adhesion quality an occurrence of adhesion defects, including week bonds, kissing bonds, and porosity voids.

8. The molding quality evaluation method according to claim 1, wherein
the step of acquiring the reference value and the step of acquiring the measurement value each include measuring a capacitance value at a plurality of locations in the molded product,
the step of deriving the measurement evaluation value includes deriving the measurement evaluation value at the plurality of locations of the molded product, and
the step of evaluating the molding quality includes evaluating a distribution of the molding quality in the molded product based on a plurality of the measurement evaluation values.

9. A molded product inspection method for inspecting the molding quality of the molded product based on an evaluation result of the molding quality evaluation method according to any one of claims 1 to 8, a strength evaluation value satisfying a required strength of the molded product being acquired in advance, the molded product inspection method comprising the steps of:
acquiring a sound evaluation value of the molded product whose molding quality has been previously evaluated to be sound;
acquiring a defective evaluation value of the molded product whose molding quality has been previously evaluated to be defective;
acquiring the measurement evaluation value derived in the molding quality evaluation method; and
determining whether an inspection has been passed, based on the acquired measurement evaluation value and strength evaluation value, wherein
the strength evaluation value is set between the sound evaluation value and the defective evaluation value.

10. A molding quality evaluation device for evaluating molding quality of a molded product containing resin, the molding quality evaluation device comprising:
a pair of electrodes placed to form a capacitor in the molded product; and
a control unit that measures a capacitance value between the pair of electrodes and evaluates the molding quality of the molded product based on the measured capacitance value, wherein
the control unit is configured to perform the steps of:
with a state of the molded product before molding being set as a reference state, acquiring a capacitance value of the molded product in the reference state as a reference value;
with a state of the molded product being set as a molding state developed over time from the reference state and being a state of the molded product during molding, acquiring the capacitance value of the molded product in the molding state as a measurement value;
deriving as a measurement evaluation value a value obtained by non-dimensionalizing the measurement value with the reference value; and
evaluating the molding quality based on the measurement evaluation value.

11. The molding quality evaluation device according to claim 10, wherein the pair of electrodes are metal thin films.

12. The molding quality evaluation device according to claim 10, wherein the pair of electrodes are provided at a plurality of locations on the molded product.

13. The molding quality evaluation device according to claim 10, further comprising:
a bagging film that accommodates the molded product inside; and
an insulating spacer that maintains an insulating state between the pair of electrodes, wherein
the pair of electrodes are a pair of electrode layers formed inside the bagging film.

14. The molding quality evaluation device according to claim 10, further comprising a bagging film that accommodates the molded product inside, wherein
the pair of electrodes are provided outside the bagging film.

15. The molding quality evaluation device according to claim 10, wherein the pair of electrodes include at least one of a metal mesh provided inside the molded product and a metal jig used during molding of the molded product.

16. A molded product inspection device for inspecting the molding quality of the molded product based on an evaluation result of the molding quality evaluation device according to any one of claims 10 to 15, the molded product inspection device comprising:
a storage unit that stores therein a strength evaluation value satisfying a required strength of the molded product; and
a control unit that determines whether the molded product satisfies the required strength, based on the strength evaluation value, wherein
the control unit is configured to perform the steps of:
acquiring a sound evaluation value of the molded product whose molding quality has been previously evaluated to be sound;
acquiring a defective evaluation value of the molded product whose molding quality has been previously evaluated to be defective;
acquiring the measurement evaluation value derived in the molding quality evaluation device; and
determining whether an inspection has been passed, based on the acquired measurement evaluation value and strength evaluation value, and
the strength evaluation value is set between the sound evaluation value and the defective evaluation value.
